# EUROPEAN PATENT APPLICATION

(11) **EP 3 225 276 A1**
(43) Date of publication of application: **04.10.2017**
(21) Application number: 15863705.8
(22) Date of filing: 19.11.2015
(51) Int. Cl.: A61M 37/00

(54) **MICRONEEDLE DEVICE**

(30) Priority: 28.11.2014 JP 2014241676
(71) Applicant: Terumo Kabushiki Kaisha, Tokyo 151-0072 (JP)
(72) Inventor: OOMORI, Sayaka, Ashigarakami-gun Kanagawa 259-0151 (JP); OOSHIMA, Hidehiko, Tokyo 163-1450 (JP)
(74) Representative: Casalonga
(86) International application number: PCT/JP2015/082552
(87) International publication number: WO 2016/084701

(57) **Abstract**

A microneedle device is provided which can stably increase a drug delivery rate.

A microneedle device includes a substrate; a microneedle projecting from the substrate and configured to be inserted into skin; and a drug coating the microneedle. An aspect ratio of a maximum width of a base of the microneedle to a height of the microneedle is equal to or greater than 2.1.

## Description

### Technical Field

The present invention relates to a microneedle device including minute projections for transdermal administration of a drug.

### Background Art

Oral administration methods and parenteral administration methods are conventionally used as drug administration methods. Oral administration methods make it easy to administer a drug. However, certain drugs may not effectively reach an affected area when they pass through the digestive system.

Examples of parenteral administration methods include methods for injection administration and methods for transdermal administration such as an ointment and an adhesive skin patch. Injection administration is effective in the respect that direct administration into a body can be performed. However, injecting a drug with an injection needle directly under the skin or into a muscle; generally involves pain and the skin or muscle may be damaged, which places a great burden on a patient.

In contrast, transdermal administration methods such as an ointment and an adhesive skin patch are easy and not cause pain, and the skin or muscle is not damaged, which can reduce a burden on a patient. However, a skin has the biological barrier function for protection against entry of foreign substances. These methods allow a drug to infiltrate the stratum corneum to a certain degree as long as the drug is lipophilic. However, a hydrophilic drug is prevented from infiltrating the stratum corneum.

Hence, a method for transdermal administration of a drug with a microneedle device including multiple minute microneedles has recently been proposed as a method for transdermal administration of a wide range of drugs (refer to, for example, Patent Literature 1).

The microneedle device allows transdermal administration of a wide range of pharmacologically active agents or the like by inserting minute needle-shaped microneedles with a height of approximately several hundred µm into the stratum corneum that prevents the infiltration of a drug and administering the drug directly into the epidermis, the dermis, or the like. Moreover, the microneedle is much smaller than an injection needle and the like; accordingly, drug administration methods using a microneedle device can reduce a burden on a patient with little pain and damage to the skin and muscle.

### Citation List

### Patent Literature

Patent Literature 1: JP 2013-248299 A

### Summary of Invention

### Technical Problem

It is difficult to adjust the insertion state when using microneedle device because the microneedles are short. The intradermal drug delivery rate may not be as expected, or may be unstable changing at every administration.

The present invention has been made to solve the above-mentioned problem, and an object thereof is to provide a microneedle device that can stably increase an intradermal drug delivery rate.

### Solution to Problem

A microneedle device includes: a substrate; a microneedle projecting from the substrate, and configured to inserted into a skin; and a drug coating the microneedle, wherein an aspect ratio of a maximum width of a base to a height of the microneedle is equal to or greater than 2.1.

### Advantageous Effects of Invention

The microneedle device configured as described above has an aspect ratio of 2.1 or greater; accordingly it is possible to improve the puncture capability of a microneedle, ensure the delivery of a drug into the skin, and stably increase a drug delivery rate.

The drug coating the microneedle is located within an area from an apex to 50% of the height of the microneedle in a height direction of the microneedle. Accordingly, the drug does not coat base part of the microneedle, which is difficult to enter the skin. Hence, the drug delivery rate can be stably increased.

The aspect ratio is equal to or less than five. Accordingly, the microneedle does not become too thin. Hence, the occurrence of breakage, deformation, and the like of the microneedle can be prevented.

### Brief Description of Drawings

Fig. 1 is a schematic perspective view showing a microneedle device according to an embodiment.
Fig. 2 is a schematic perspective view showing a microneedle of the microneedle device according to the embodiment of Fig.1.
Fig. 3 is a schematic cross-sectional view for explaining the step of coating the microneedles with a drug by a dipping method.
Fig. 4 is a picture taken when microneedles of Examples 1 and 2 were microscopically observed.
Fig. 5 is a picture taken when microneedles of Examples 3 and 4 were microscopically observed.
Fig. 6 is a picture taken when microneedles of Examples 5 and 6 were microscopically observed.
Fig. 7 is a picture taken when microneedles of Comparative Examples 1 and 2 were microscopically observed.
Fig. 8 is a perspective view showing a modification of the microneedle device.
Fig. 9 is a perspective view showing another modification of the microneedle device.
Fig. 10 is a perspective view showing still another modification of the microneedle device.

### Description of Embodiments

An embodiment of the present invention is described hereinafter with reference to the drawings. The dimensional ratios of the drawings may be different from the actual ratios, exaggerated for convenience of description.

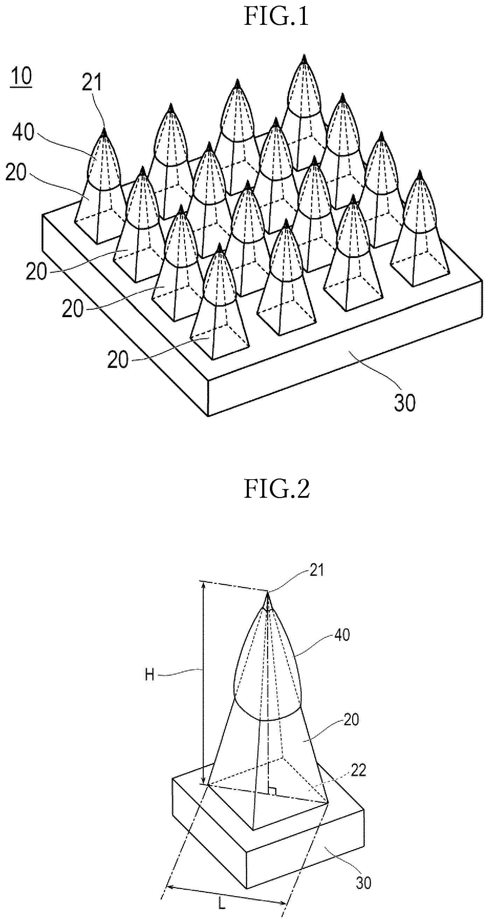

A microneedle device 10 according to the embodiment is a device for transdermal administration of a drug being a pharmacologically active agent into a body by being adhered to the skin. The microneedle device 10 includes a substrate 30, microneedles 20 formed on the substrate 30, and a drug 40 coating the microneedles 20 as shown in Fig. 1.

The microneedle 20 is formed in the shape of a regular square pyramid, and is formed in such a manner as to be tapered from a base 22 on the substrate 30 toward an apex 21. The shape of the microneedle is not limited as long as the microneedle protrudes in such a manner that the entire microneedle is tapered toward the direction of the apex. The microneedle may be formed in, for example, the shape of another pyramid having a different number of sides, or the shape of a cone.

Moreover, a distal end shape of the apex 21 of the microneedle 20 is not especially limited as long as the microneedle can be inserted into the skin to provide a through-hole in the stratum corneum. Therefore, the apex 21 of the microneedle 20 may not be formed in such a perfect apex as shown in Figs. 1 and 2, or may be formed into a curved surface or flat surface to a degree that allows insertion of the microneedle into the skin.

A height H of the microneedle 20 is preferably one that allows insertion of the microneedle into the skin and administration of the drug 40 deeper than the stratum corneum without reaching a nerve in the dermis. The height H of the microneedle 20 is, for example, within a range from 10 µm to 1500 µm. If the height H of the microneedle 20 is too short, it may become difficult to stably insert the microneedle 20 into the skin. If the height H of the microneedle 20 is too long, the microneedle 20 may reach a nerve in the dermis to cause pain upon administration of the drug 40. Even if the height H exceeds 1000 µm (1 mm), the microneedle 20 is referred to as the microneedle as long as the object can be achieved. Therefore, the microneedle device 10 including microneedles 20 with height H that exceeds 1000 µm (1 mm) is also referred to as the microneedle device as long as the object can be achieved.

The number per unit area of the microneedles 20 to be formed on the substrate 30 is selectable, as appropriate, according to the application and the like of the microneedle device 10. For example, it is within a range from one needle/cm² to 10000 needles/cm².

The aspect ratio being a ratio of a maximum width L of the base 22 of the microneedle 20 to the height H from the base 22 to the apex 21 is preferably equal to or greater than 2.1. The height H here indicates a height in a direction perpendicular to the base 22 (or the substrate 30). The maximum width L indicates the width of a portion, which has the largest width, of the base 22. Therefore, the maximum width L of a regular square pyramid is the length of a diagonal line of the base 22. The aspect ratio is equal to or greater than 2.1 to make the microneedle 20 sharp and accordingly make it easier to insert the microneedle 20 into the skin and administer the drug 40 deeper than the stratum corneum. Hence, the delivery rate of the drug 40 can be stabilized at a high rate. Moreover, the aspect ratio is preferably equal to or less than five. If the aspect ratio exceeds five, it may become difficult to maintain the strength of the thin microneedle 20.

The maximum width L of the base 22 of the microneedle 20 can be set, as appropriate, according to the height H of the microneedle 20 in such a manner that the aspect ratio is equal to or greater than 2.1.

The drug 40 is preferably retained more on the apex side than the base side of the microneedle 20. An area, which is coated with the drug 40, of the microneedle 20 in the height direction is within an area from the apex 21 to 50% of the height H of the microneedle 20, more preferably, within an area from the apex 21 to 40% of the height H, still more preferably, within an area from the apex 21 to 30% of the height H. The amount of the drug 40 retained on the apex side of the microneedle 20 is increased in this manner; therefore, the amount of the drug 40 that reaches deeper than the stratum corneum can be increased, while the amount of the drug 40 that is attached to the surface of the stratum corneum and is not delivered into the skin can be reduced. The positon to retain the drug 40 is not limited to the above-mentioned area, and the drug 40 may be retained on the base side of microneedle 20.

The constituent material of the microneedle 20 is not especially limited as long as it can form the microneedle 20. A material typically used for a microneedle device 10 can be applied.

Specifically, examples of the constituent material of the microneedle 20 include an inorganic material and a resin material. Examples of the inorganic material include metal such as silicon, silicon dioxide, ceramic, stainless steel, iron, aluminum, titan, nickel, molybdenum, chrome, cobalt, copper, lead, niobium, tantalum, zirconium, tin, gold, and silver.

Examples of the resin material include biodegradable polymers such as polylactic acid, polyglycolide, polylactide-co-polyglycolide, pullulan, caprolacton, polyurethane, and polyanhydride. Moreover, examples of the resin material also include non-biodegradable polymers such as polycarbonate, polymethacrylic acid, ethylene vinyl acetate, polytetrafluoroethylene, polyoxymethylene, cycloolefin polymer (COP), and cycloolefin copolymer (COC).

Among the above-mentioned materials, the microneedle 20 preferably includes a hydrophobic material exhibiting liquid repellency. If the microneedle includes a material having high wettability and low liquid repellency, it may be impossible to prevent the travel of the drug flowing in the substrate direction; accordingly, the vicinity of the base part of the microneedle may be coated with a large amount of the drug. In contrast, liquid repellency is added to the surface of the microneedle 20, which excellently allows the restriction of the travel of the drug 40 flowing from the apex 21 to the substrate direction when the drug 40 coats the microneedle 20.

Specifically, examples of the material having liquid repellency include polycarbonate, polymethacrylic acid, ethylene vinyl acetate, polytetrafluoroethylene, polyoxymethylene, cycloolefin polymer (COP), and cycloolefin copolymer (COC). Moreover, additional surface treatment such as fluorinating may be performed to cause the microneedle 20 to have liquid repellency.

Moreover, surface treatment that increases wettability may be performed on the surface of the microneedle 20 so as to have a thin coating of the drug 40 on the surface. Examples of the treatment to increase wettability include a process to make the surface rough and plasma processing.

The microneedle 20 may be formed separately from the substrate 30, but is preferably formed integrally with the substrate 30. A specific formation method is described below.

The substrate 30 holds the base 22 of the microneedle 20 in such a manner that a plurality of the microneedles 20 can be collectively inserted into the skin. The substrate 30 may be separate from the microneedles 20, but is usually formed integrally with the microneedles 20 as described above.

The thickness of the substrate 30 is not especially limited as long as the substrate 30 can hold the microneedles 20 and has some strength that allows the microneedles 20 to be inserted into the skin. The thickness of the substrate 30 is, for example, within a range from 10 µm to 1500 µm.

The size, shape, and the like of the substrate 30 are not especially limited, and are selectable, as appropriate, according to the application and the like of the microneedle device 10.

The above-mentioned material used for the microneedle 20 can also be used as the constituent material of the substrate 30. Alternatively, the constituent material of the substrate 30 may be different from the material used for the microneedle 20.

A method for fabricating the microneedle device 10 according to the embodiment described above is not especially limited as long as the microneedles 20 can be formed on the substrate 30. A method similar to a general microneedle fabricating method can be used. Specifically, examples of the fabricating method include etching, electrical discharge machining, sandblasting, laser processing, hot embossing, machining, plating, injection molding, imprinting, and photolithography.

A method for fabricating the microneedle device 10 by imprinting is specifically described as an example. First, a stamper, which is a mold including depressions matching the shape of the microneedle 20, and a base material including the material of the microneedle 20 such as a thermoplastic resin are prepared. Next, the base material is heated to be softened. The stamper is pressed against the surface of the softened base material. The base material is then cooled to be hardened. The stamper is separated from the base material. The microneedles 20 of the shape matching the depression are transferred onto the surface of the base material to obtain the microneedle device 10.

A known stamper that is generally used for the fabrication of a microneedle device can be used as the stamper. The stamper may be, for example, one formed by, for example, machining the surface of a metal base material or the like. Alternatively, the stamper may be formed by, for example, etching and cutting a metal base material or the like, forming a master plate of the microneedle device 10, and, for example, plating the master plate.

Examples of a method for coating the microneedles 20 with the drug 40 include an inkjet method for spraying and applying the drug 40 via a nozzle or the like from a position away from the microneedles 20, a dispenser method for applying the drug 40 to the microneedles 20 via a nozzle or the like, and a dipping method (dipping method) for dipping the microneedles 20 in the drug to attach the drug thereto. Among these methods, the dipping method is effective to thoroughly coat a distal end region of the microneedle 20 with the drug 40.

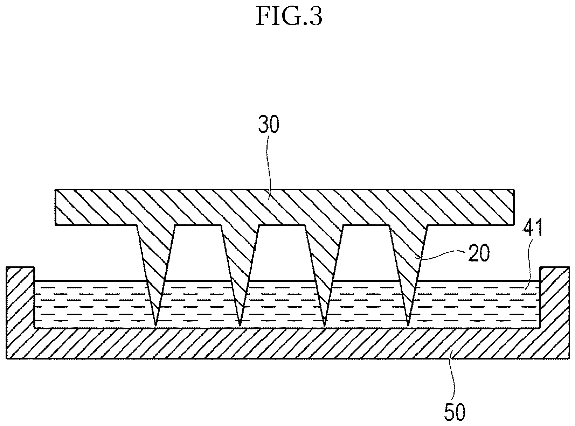

In the dipping method, the microneedles 20 are dipped in a solution 41 containing the drug in a container 50 with the apices 21 of the microneedles 20 down to coat the microneedles 20 as shown in Fig. 3. The microneedles 20 are subsequently raised to volatize the solvent of the solution 41. Accordingly, a layer of the drug 40 is formed on the microneedles 20. At this point in time, a depth to which the microneedles 20 are dipped is adjusted to enable the selective coating of the drug 40 onto the surface on the apex 21 side of the microneedle 20.

Next, the operation of the microneedle device 10 according to this embodiment is described.

The drug 40 is administered using the microneedle device 10 by inserting the microneedles 20 coated with the drug 40 into the stratum corneum of the skin, opening through-holes in the stratum corneum, and causing the microneedles 20 to reach the epidermis or the like deeper than the stratum corneum through the through-holes. The aspect ratio of the microneedle device 10 according to the embodiment is equal to or greater than 2.1. Accordingly, the puncture capability is high, which makes it easier for the microneedles 20 to reach the epidermis or the like deeper than the stratum corneum. Hence, the drug 40 can be delivered without waste by causing the drug 40 to efficiently reach deeper than the stratum corneum. Therefore, the intradermal drug delivery rate can be stably increased. The stable increase in intradermal drug delivery rate makes the drug administration rate difficult to change at every administration. Accordingly, a desired amount of the drug can be always administered. The drug can be effectively administered, and safety is also increased.

Moreover, the area, which is coated with the drug 40, of the microneedle 20 in the height direction is within the area from the apex 21 to 50% of the height H of the microneedle 20. Accordingly, the amount of the drug 40 that is attached to the stratum corneum and is not delivered into the skin upon the insertion of the microneedles can be further reduced, while the amount of the drug 40 that reaches under the layer of the stratum corneum can be further increased.

Moreover, since the aspect ratio is equal to or less than five, the microneedle 20 does not become too thin. Accordingly, it is possible to prevent the occurrence of breakage, deformation, and the like of the microneedle 20, and stably increase the intradermal drug delivery rate.

### Examples

Microneedle devices including drug-coated microneedles were produced to carry out a test to examine drug delivery properties using rats.

### Examples 1 and 2

Microneedle devices including, on a substrate, 25 (5 x 5) regular square pyramid-shaped microneedles (aspect ratio: 2.12) measuring 167 µm per side of the base and 500 µm in height were produced by imprinting using cyclic polyolefin copolymer as a constituent material.

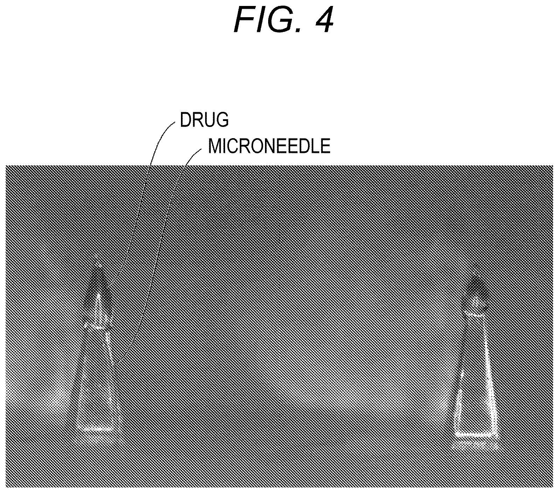

The drug that coated the microneedles was lidocaine hydrochloride, and was mixed with a binder (binder), a fluorescent dye to observe the state of the drug on the microneedles, and India ink for visualization at the time of production. The India ink contained carbon, glue, and water. Polyvinylpyrrolidone (PVP) was used as the binder. Dextran-tetramethylrhodamine (TMR) (10000 molecular weight) was used as the fluorescent dye. The concentration of dissolved lidocaine hydrochloride before coating was 50%, and the concentration of dissolved dextran-tetramethylrhodamine before coating was 0.1%. Ethanol was used as a solvent. The microneedles were coated by the dipping method with the above-mentioned drug. The solvent was volatized to obtain microneedle devices according to Examples 1 and 2. Fig. 4 shows a picture taken to microscopically observe part of the microneedle devices according to Examples 1 and 2.

The time for attachment to the skin of an assessment test described below was set at five minutes in Example 1, and the attachment time of the assessment test described below was set at 60 minutes in Example 2.

### Examples 3 and 4

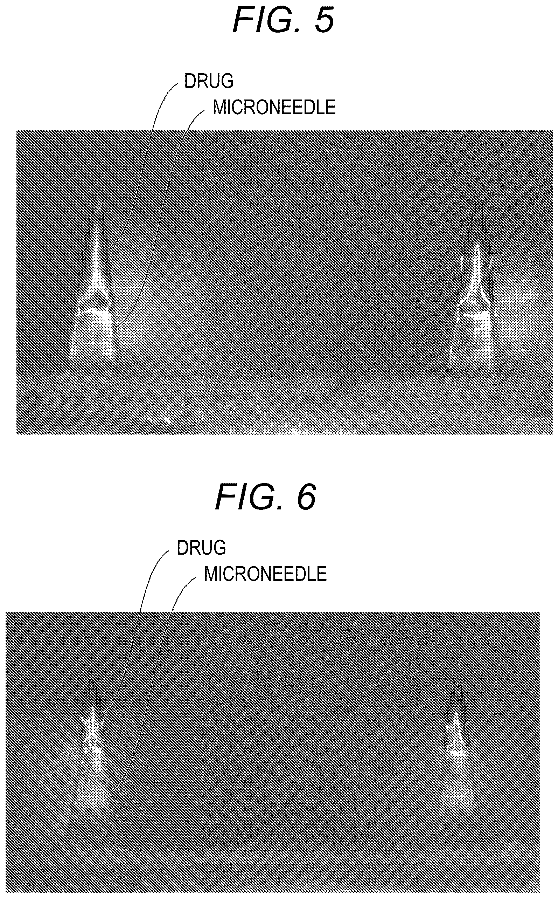

25 (5 x 5) microneedles were formed on a substrate using a similar material and method to Examples 1 and 2 described above. Surface treatment was performed to thinly coat the microneedles with the drug. The entire surface of the microneedles was coated by the dipping method with the drug, using the drug, the binder, the fluorescent dye, and the India ink that were formulated as in Examples 1 and 2. The drug was then volatized to obtain a microneedle device according to each of Examples 3 and 4. The attachment time of the assessment test described below was set at five minutes in Example 3, and the attachment time of the assessment test described below was set at 60 minutes in Example 4. Fig. 5 shows a picture taken to microscopically observe part of the microneedle devices according to Examples 3 and 4.

### Examples 5 and 6

25 (5 x 5) microneedles were formed on a substrate using a similar material and method to Examples 1 and 2 described above. Surface treatment was performed to thinly coat the microneedles with the drug. The microneedles were coated by the dipping method with the drug so as to coat approximately 150 µm from the apex side, using the drug, the binder, the fluorescent dye, and the India ink that were formulated as in Examples 1 and 2. The drug was then dried to obtain a microneedle device according to each of Examples 5 and 6. The attachment time of the assessment test described below was set at five minutes in Example 5, and the attachment time of the assessment test described below was set at 60 minutes in Example 6. Fig. 6 shows a picture taken to microscopically observe part of the microneedle devices according to Examples 5 and 6. Comparative Examples 1 and 2

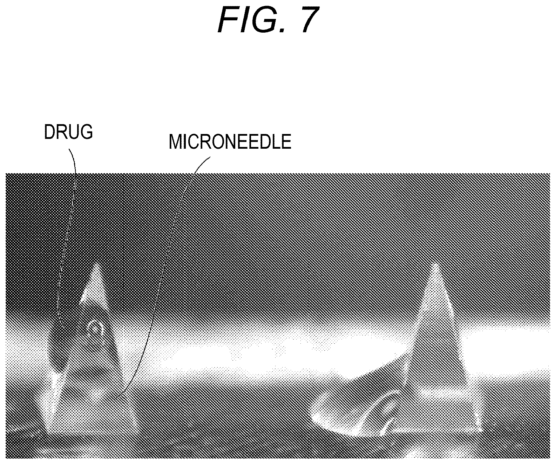

Microneedle devices including, on a substrate, 25 (5 x 5) regular square pyramid-shaped microneedles (aspect ratio: 1.41) measuring 250 µm per side of the base and 500 µm in height were produced by imprinting. Next, the microneedles were coated by the dipping method with the drug, using the drug, the binder, the fluorescent dye, and the India ink that were formulated as in Examples 1 and 2. The drug was dried to obtain a microneedle device according to each of Comparative Examples 1 and 2. The attachment time of the assessment test described below was set at five minutes in Comparative Example 1, and the attachment time of the assessment test described below was set at 60 minutes in Comparative Example 2. Fig. 7 shows a picture taken to microscopically observe part of the microneedle devices according to Comparative Examples 1 and 2.

### [Assessment Test]

The microneedles were inserted into the lumbar skin of a rat whose hair was removed under anesthesia. After passage of a predetermined attachment time, the microneedle device was removed from the skin. The amount of lidocaine hydrochloride remaining on the needles, the amount remaining on the surface of the skin, and the intradermal delivery amount were measured by high-performance liquid chromatography (HPLC) to calculate an intradermal delivery rate.

### [Insertion of Microneedles and Sampling]

(1) The back of the rat under anesthesia (isoflurane by inhalation, 1 to 4%) was shaved with a shaver (ES795PP made by Panasonic). A hair removal cream (Veet hair removal cream by Reckitt Benckiser) was applied for five minutes to remove the hair.
(2) The hair removal cream was washed away and the back of the rat was wiped with acetone.
(3) The skin surface was marked in a width of 20 mm and elongated to 25 mm. After insertion of the microneedles under conditions shown in Table 2, the microneedle device was fixed to the skin with a tape (Dermicel by Johnson & Johnson), and was left attached for a predetermined time.
(4) The microneedles were removed from the skin. Cotton balls whose weight was measured in advance were moistened with phosphate-buffered saline (PBS). The administration site was wiped three times with the cotton balls and then once with a dry cotton ball.
(5) The skin at the administration site was extracted with a diameter of 2 cm.

### [Measurements of Drug Delivery Properties]

(1) The microneedles after being inserted into the rat were soaked in 1 mL of distilled water for one night to take a needle remaining amount sample of HPLC.
(2) The weights of PBS of the four cotton balls that wiped the administration site were measured. 3 mL of distilled water was then added thereto and left for one night to take a skin surface remaining amount sample of HPLC.
(3) The weight of the extracted skin was measured. The extracted skin was cut by scissors into pieces of a square millimeter each. 5 mL of normal saline was added thereto for homogenization under ice cooling with a homogenizer (Polytron). Accordingly, skin extract was taken. 200 µL of supernatant of the skin extract was taken into a microtube. Ice-cooled acetonitrile (500 µL) was added therein. After mixing, the microtube underwent centrifugation (10000 rpm, 5 min) to remove protein. 20 mM potassium phosphate buffer (pH 5.8) and the supernatant of the skin extract, 200 µL each, were added into Centricut (W-MO 0.2 µm by Kurabo). After mixing, Centricut underwent centrifugation (5000 G, 5 min) to take an intradermal delivery amount sample of HPLC.
(4) Lidocaine hydrochloride of each sample was measured by HPLC. Table 1 shows the conditions of HPLC.

**[Table 1]**

| Item | Conditions of HPLC |
|---|---|
| Mobile phase | 20 mM potassium phosphate buffer (pH 5.8)/ acetonitrile (65:35) |
| Flow rate | 0.5 mL/min |
| Measurement time | 20 min |
| Sample amount | 10 µL |
| Column | Inner diameter 3.00 mm, Length 150 mm, Particle size 5 µm (Unison, US-C18), Guard column (Unison, US-C18(C-18)) |
| Column temperature | 40°C |
| Detection | UV detector wavelength 210 nm |

(5) The amount remaining on the needles, the amount remaining on the skin surface, the intradermal delivery amount were calculated from measurement values of the concentrations of lidocaine hydrochloride of the needle remaining amount sample, the skin surface remaining amount sample, and the skin extract. The rates of the needle remaining amount, the skin surface remaining amount, and the intradermal delivery amount with respect to a total thereof were respectively calculated as a needle remaining amount rate, a skin surface remaining amount rate, and an intradermal delivery rate.

### [Test Results]

Table 2 shows the needle remaining amount rate, the skin surface remaining amount rate, the intradermal delivery rate of lidocaine hydrochloride in Examples and Comparative Examples together with the test conditions.

**[Table 2]**

| | Aspect ratio | Drug coating conditions | Attachment time | Needle remaining amount rate | Skin surface remaining amount rate | Intradermal delivery rate |
|---|---|---|---|---|---|---|
| | | | (min) | (%) | (%) | (%) |
| Example 1 | 2.12 | Entire surface coated | 5 | 25 | 44 | 31 |
| | | No surface treatment | | | | |
| Example 2 | 2.12 | Entire surface coated | 60 | 36 | 35 | 29 |
| | | No surface treatment | | | | |
| Example 3 | 2.12 | Entire surface coated | 5 | 19 | 63 | 17 |
| | | Surface treated | | | | |
| Example 4 | 2.12 | Entire surface coated | 60 | 26 | 34 | 40 |
| | | Surface treated | | | | |
| Example 5 | 2.12 | Only the apex side coated | 5 | 36 | 28 | 36 |
| | | Surface treated | | | | |
| Example 6 | 2.12 | Only the apex side coated | 60 | 7 | 34 | 59 |
| | | Surface treated | | | | |
| Comparative example 1 | 1.41 | Entire surface coated | 5 | 28 | 65 | 7 |
| | | No surface treatment | | | | |
| Comparative example 2 | 1.41 | Entire surface coated | 60 | 62 | 31 | 7 |
| | | No surface treatment | | | | |

As shown in Table 2, it has been confirmed from comparisons of Examples 1 to 6 and Comparative Examples 1 and 2 that the intradermal drug delivery rate is higher in Examples 1 to 6 where the aspect ratio is equal to or greater than 2.1 than in Comparative Examples 1 and 2 where the aspect ratio is less than 2.1.

Moreover, it has been confirmed from comparisons of Examples 5 and 6 and Examples 3 and 4 that when the aspect ratios are the same, the intradermal delivery rate is higher in the case where only the distal end region of the microneedles was coated with the drug (Examples 5 and 6) than in the case where the microneedles were entirely coated with the drug (Examples 3 and 4).

Moreover, it has been confirmed from Examples 3 to 6 that the intradermal drug delivery rate is increased as the attachment time is increased.

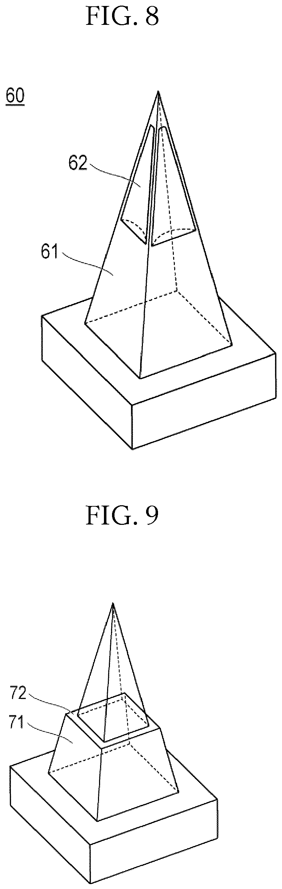

The present invention is not limited only to the above-mentioned embodiment. Various modifications can be made by a person skilled in the art within the technical ideas of the present invention. For example, indentations 62 to retain a drug may be formed on a microneedle 61 as shown in Fig. 8. Consequently, the amount of the drug that coats the basal part can be reduced. Accordingly, the drug can be efficiently administered.

Moreover, a step 72 may be formed on a microneedle 71 in such a manner that the apex side is thinner as shown in Fig. 9. Consequently, the drug retaining amount can be increased on the surface on the apex side of the microneedle 71 with respect to the step 72, while the drug retaining amount at the base part can be reduced. Accordingly, the drug can be efficiently administered.

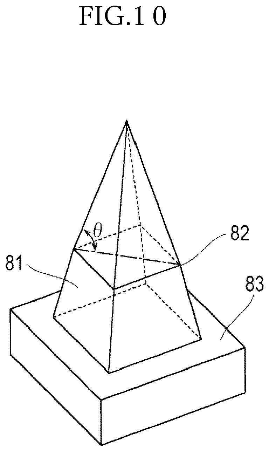

Moreover, as shown in Fig. 10, a changing portion 82 where an inclination angle θ of the apex side with respect to a substrate 83 changes so as to be smaller than the base side may be formed in a microneedle 81. Consequently, the drug retaining amount on the surface on the apex side of the microneedle 81 can be increased, while the drug retaining amount on the base side can be reduced. Accordingly, the drug can be efficiently administered. Furthermore, a step is not formed to facilitate the insertion of the microneedle 81 up to the vicinity of the base part. Accordingly, the drug can be efficiently administered. Moreover, the microneedle may be formed in such a manner that the inclination angle of the surface of the microneedle with respect to the substrate is gradually reduced toward the apex side. Consequently, the surface of the microneedle becomes smooth, which makes even easier to insert the microneedle. Accordingly, the drug can be efficiently administered.

Furthermore, the present application is based on Japanese Patent Application No. 2014-241676 filed on November 28, 2014. The entire content of the disclosure is incorporated herein as a reference.

### Reference Numeral List

- 10: Microneedle device
- 20, 61, 71, 81: Microneedle
- 21: Apex
- 22: Base
- 30, 83: Substrate
- 40: Drug
- 83: Substrate

## Claims

1. A microneedle device comprising:
a substrate;
a microneedle projecting from the substrate and configured to be inserted into skin; and
a drug coating the microneedle, wherein
an aspect ratio of a maximum width of a base of the microneedle to a height of the microneedle is equal to or greater than 2.1.

2. The microneedle device according to claim 1, wherein the drug coating the microneedle is located entirely within a region extending from an apex of the microneedle to 50% of the height of the microneedle in a height direction of the microneedle.

3. The microneedle device according to claim 1 or 2, wherein the aspect ratio is equal to or less than five.
